# EUROPEAN PATENT APPLICATION

(11) **EP 2 578 559 A1**
(43) Date of publication of application: **10.04.2013**
(21) Application number: 11306306.9
(22) Date of filing: 07.10.2011
(51) Int. Cl.: C07C 11/09, C12P 13/08, C12P 13/00, C12N 9/88

(54) **Process for producing isobutene from isobutylamine**

(71) Applicant: Metabolic Explorer, 63360 Saint Beauzire (FR)
(72) Inventor: Boisart, Cédric, 63360 GERZAT (FR)
(74) Representative: Regimbeau

(57) **Abstract**

The present invention concerns a new process for the sustainable chemical synthesis of isobutene, using isobutylamine obtained by fermentation of a recombinant microorganism as starting material, based on a chemical conversion of an amine to an olefin (alkene).

## Description

### INTRODUCTION

The present invention concerns a new process for the sustainable chemical synthesis of isobutene, using isobutylamine obtained by fermentation of a recombinant microorganism as starting material, based on a chemical conversion of an amine to an olefin (alkene).

Isobutene, also called isobutylene, 2-Methylpropene or 2-methylpropylene (CAS number 115-11-7) is a highly flammable gas and presents an explosion danger. Its molecular formula is C4H8. It belongs to the group of alkenes (also called olefins) which are unsaturated chemical compounds containing at least one carbon-to-carbon double bond.

Isobutene is a chemical component widely used in industry, as an intermediate in the production of a variety of products. It is reacted with methanol and ethanol in the manufacture of the gasoline oxygenates methyl tert-butyl ether (MTBE) and ethyl tert-butyl ether (ETBE), respectively. Isobutene is also used in the production of methacrolein and methyl methacrylate (MMA). Polymerization of isobutene produces butyl rubber (polyisobutylene). Antioxidants such as butylated hydroxytoluene (BHT) and butylated hydroxyanisole (BHA) are produced by Friedel-Crafts alkylation of phenols using isobutene.

Isobutene can be isolated from refinery streams by reaction with sulfuric acid, but the most common industrial method for its production is by catalytic dehydrogenation of isobutane as starting material.

### TECHNICAL BACKGROUND OF THE INVENTION

The patent application WO2010001078 discloses a process for the preparation of isobutene from 3-hydroxy-3-methylbutyrate by enzymatic conversion with Mevalonate DiPhosphate decarboxylase.

The patent application WO20100992010 describes a method of production of isobutene from C4 and/or C5 alcohol, and more particularly isobutanol, by dehydrogenation.

The patent application EP 1 944 282 describes a process for the production of isobutene by dehydrating tertiary butanol (TBA) using an alumina catalyst, a long-term stable catalyst allowing a stable process.

The patent application US 2009/0069615 describes a process for the production of isobutene by dehydrating tertiary butanol (TBA) using a solid superacid catalyst.

The patent application US 2011/0118523 describes a process for preparing isobutene by cleavage of methyl tert-butyl ether (MTBE), from MTBE-containing mixtures as starting material.

In the art, nothing is known about the preparation of isobutene from isobutylamine. The present invention is related to the production of isbutylamine by fermentation, from an inexpensive source of carbon (sugar). The present invention is also related to the conversion of said bio-produced isobutylamine into isobutene. This chemical conversion is based on the conversion of amines into olefins, and in particular to the conversion of isobutylamine into isobutene.

In the invention the conversion starting material, isobutylamine, is obtained by fermentation of a recombinant microorganism. The advantages of fermentative production of isobutylamine are in particular low cost, human safety and environmental protection and sustainability of the process.

In the prior art, no document relates to a fermentative production of isobutylamine.

Patent US7851188 describes a metabolic pathway for the production of isobutanol, wherein isobutylamine is an intermediate product in the biosynthesis pathway of isobutanol. In this patent, it is not suggested that the pathway could be used for isobutylamine or isobutene production, in particular no experimental data is given.

In the present invention, a novel method is given to produce isobutene with a combination of a first fermentation step for the production of isobutylamine, and a second chemical step for the conversion of isobutylamine into isobutene.

### BRIEF DESCRIPTION OF THE INVENTION

The present invention is related to a process for producing isobutene comprising a first step of production of isobutylamine by fermentation with a recombinant microorganism producing L-valine and expressing an exogenous gene coding for the enzyme having L-valine decarboxylase activity and a second step of chemical conversion of isobutylamine into isobutene.

The present invention is also related to a process for producing isobutylamine by fermentation, comprising:
- cultivating a recombinant microorganism producing valine in a culture medium comprising a simple source of carbon, and
- recovering the produced isobutylamine,
wherein the recombinant microorganism expresses en exogenous gene coding for an enzyme having valine decarboxylase activity.

In a specific embodiment, the exogenous gene coding for the enzyme having L-valine decarboxylase activity is the gene *vlmD* from *Streptomyces viridifaciens.*

After recovery and purification of the isobutylamine from the culture medium, several chemical reactions can be carried out in the aim to convert isobutylamine into isobutene, in particular the following reactions:
- a diazotation reaction, in presence of nitrous acid and sulphuric acid;
- a Cope reaction, in presence of m-chloroperbenzoic acid;
- a reaction in presence of pyrylium salts;
- a reaction of Hofmann elimination, in presence of methyl iodide.

Each chemical reaction presents advantages and disadvantages, and the man skilled in the art will be able to choose which reaction is the best to carry out, at the light of the present specification.

For all the chemical reactions described above, a further step of condensating the gaseous isobutene in a cold trap is planned.

The present invention is also related to a genetically modified microorganism overexpressing at least one endogenous gene involved in the L-valine biosynthesis pathway and expressing a gene coding for a L-valine decarboxylase.

### DETAILED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

All publications, patents and patent applications cited herein, whether supra or infra, are hereby incorporated by reference in their entirety. However, publications mentioned herein are cited for the purpose of describing and disclosing the protocols, reagents and vectors that are reported in the publications and that might be used in connection with the invention. Nothing herein is to be construed as an admission that the invention is not entitled to antedate such disclosure by virtue of prior invention.

Furthermore, the practice of the present invention employs, unless otherwise indicated, conventional microbiological and molecular biological techniques within the skill of the art. Such techniques are well known to the skilled worker, and are explained fully in the literature. See, for example, Prescott *et al.* (1999) and Sambrook *et al.* (1989) (2001).

It must be noted that as used herein and in the appended claims, the singular forms "a", "an", and "the" include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any materials and methods similar or equivalent to those described herein can be used to practice or test the present invention, the preferred materials and methods are now described.

In the claims that follow and in the description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

### Definitions

The term "isobutylamine" is used to designate 2-methylpropan-1-amine. Isobutylamine with CAS number 78-81-9 is an amine with the formula (CH3)2CHCH2NH2, and occurs as a colorless liquid. It is one of the four isomeric amines of butane.

The term "isobutene" is used to designate isobutylene or 2-methyl-propene. Isobutylene is an olefin with the formula C4H8.

The term "olefin" means alkene or olefine. It is an unsaturated chemical compound containing at least one carbon-to-carbon double bond.

The term "microorganism", as used herein, refers to a bacterium, yeast or fungus which are not modified artificially.

The term "microorganism producing valine" refers to a microorganism which possesses in its genome the genes involved in valine biosynthesis coding for acetolactate synthase, keto-acid reductoisomerase, dihydroxy-acid dehydratase and branched chain amino acid transaminase.

The term "recombinant microorganism" or "genetically modified microorganism", as used herein, refers to a microorganism genetically modified or genetically engineered. It means, according to the usual meaning of these terms, that the microorganism of the invention is not found in nature and is modified either by introduction or by deletion or by modification of genetic elements. It can also be transformed by forcing the development and evolution of new metabolic pathways in combining directed mutagenesis and evolution under specific selection pressure (see for instance WO 2004/076659).

A microorganism may be modified to express exogenous genes if these genes are introduced into the microorganism with all the elements allowing their expression in the host microorganism. A microorganism may be modified to modulate the expression level of an endogenous gene. The modification or "transformation" of microorganisms with exogenous DNA is a routine task for those skilled in the art.

The term "endogenous gene" means that the gene was present in the microorganism before any genetic modification, in the wild-type strain. Endogenous genes may be overexpressed by introducing heterologous sequences in addition to, or to replace endogenous regulatory elements, or by introducing one or more supplementary copies of the gene into the chromosome or a plasmid. Endogenous genes may also be modified to modulate their expression and/or activity. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, down regulate and/or lower the activity of the endogenous gene product. Another way to enhance expression of endogenous genes is to introduce one or more supplementary copies of the gene onto the chromosome or a plasmid.

The term "exogenous gene" means that the gene was introduced into a microorganism, by means well known by the man skilled in the art whereas this gene is not naturally occurring in the microorganism. Exogenous genes can be heterologous or not. A microorganism can express exogenous genes if these genes are introduced into the microorganism with all the elements allowing their expression in the host microorganism. Transforming microorganisms with exogenous DNA is a routine task for the man skilled in the art. Exogenous genes may be integrated into the host chromosome, or be expressed extra-chromosomally by plasmids or vectors. A variety of plasmids, which differ with respect to their origin of replication and their copy number in the cell, are all known in the art. These genes may be heterologous or homologous.

The term "heterologous gene" means that the gene is derived from a species of microorganism different from the recipient microorganism that expresses it. It refers to a gene which is not naturally occurring in the microorganism. All genes are referenced with their common names and with references that give access to their nucleotidic sequences on the website http://www.ncbi.nlm.nih.gov/gene.

The man skilled in the art knows different means to modulate, and in particular up-regulate, the expression of endogenous genes. For example, a way to enhance expression of endogenous genes is to introduce one or more supplementary copies of the gene onto the chromosome or a plasmid.

Another way is to replace the endogenous promoter of a gene with a stronger promoter. These promoters may be homologous or heterologous. It is well within the ability of the person skilled in the art to select appropriate promoters, for example, the promoters Ptrc, Ptac, Plac or the lambda promoter cl are widely used.

Finally, the sequence of exogenous or heterologous gene may be adapted for its expression in the host microorganism. Indeed, the man skilled in the art knows the notion of codon usage bias and how to adapt nucleic sequence for a particular codon usage bias without modifying the translated protein.

The term 'overexpression' means in this context that the expression of a gene or an enzyme is increased compared to a non modified microorganism. Increase of expression of an enzyme is obtained by the increase of the expression of a gene encoding said enzyme.

The 'activity' of an enzyme is used interchangeably with the term 'function' and designates, in the context of the invention, the reaction that is catalyzed by the enzyme.

The terms "encoding" or "coding" refer to the process by which a polynucleotide, through the mechanisms of transcription and translation, produces an amino-acid sequence.

The gene(s) encoding the enzyme(s) can be exogenous or endogenous.

"Attenuation" of genes may be achieved by means and methods known to the man skilled in the art and contains gene deletion by homologous recombination, gene attenuation by insertion of an external element into the gene or gene expression under a weak promoter. The man skilled in the art knows a variety of promoters which exhibit different strength and which promoter to use for a weak genetic expression.

The "fermentation" is generally conducted in fermenters with an appropriate culture medium adapted to the microorganism being used, containing at least one simple carbon source, and if necessary co-substrates.

An "appropriate culture medium" designates a medium (e.g., a sterile, liquid media) comprising nutrients essential or beneficial to the maintenance and/or growth of the cell such as carbon sources or carbon substrates, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids and vitamins.

As an example of known culture media for *E. coli,* the culture medium can be of identical or similar composition to an M9 medium (Anderson, 1946), an M63 medium (Miller, 1992); or a medium such as defined by Schaefer *et al.* (1999).

As another example of culture medium for *C. glutamicum,* the culture medium can be of identical or similar composition to BMCG medium (Liebl *et al.,* 1989) or to a medium such as described by Riedel *et al.* (2001).

The term "carbon source" or "carbon substrate" or "source of carbon" according to the present invention denotes any source of carbon that can be used by those skilled in the art to support the normal growth of a micro-organism, including hexoses (such as glucose, galactose or lactose), pentoses, monosaccharides, oligosaccharides, disaccharides (such as sucrose, cellobiose or maltose), molasses, starch or its derivatives, hemicelluloses and combinations thereof. An especially preferred simple carbon source is glucose. Another preferred simple carbon source is sucrose.

The present invention is related to a method for producing isobutene comprising:
- a first step of production of isobutylamine by fermentation in a culture medium comprising a simple source of carbon of a recombinant microorganism producing L-valine, and
- a second step of conversion of isobutylamine into isobutene by chemical conversion,
wherein the recombinant microorganism expresses an exogenous gene coding for an enzyme having L-valine decarboxylase activity.

### Isobutylamine production

The present invention relates to a process for the chemical synthesis of isobutene, using isobutylamine obtained by fermentation of a recombinant microorganism as starting material, based on a chemical conversion of an amine to an olefin.

In the invention, the starting material isobutylamine is obtained by fermentative production with a recombinant microorganism expressing a L-valine decarboxylase, in a culture medium comprising a source of carbone.

Said recombinant microorganism is preferably transformed for the expression of a heterologous gene coding for L-valine decarboxylase. Several microorganisms exhibit valine decarboxylase activity such as *Bacillus sphaericus* (Bast *et al.,* 1971), *Proteus vulgaris* (Haughton *et al.,* 1961) or *Streptomyces species* (*Garg et al., 2002*). Among these candidates having L-valine decarboxylase activity, the gene encoding this enzyme has been identified for only one species: *Streptomyces viridifaciens.* In a preferred aspect of the invention, said exogenous gene is *vlmD* from *Streptomyces viridifaciens.* In a more preferred aspect of the invention, the sequence of the *vlmD* gene is adapted for its expression in the host microorganism. Indeed, the man skilled in the art knows the notion of codon usage bias and how to adapt nucleic sequences for a particular codon usage bias without modifying the translated protein.

Nevertheless the man skilled in the art will be able to obtain a gene encoding the L-valine decarboxylase enzyme from different microorganisms exhibiting said enzymatic activity.

In a preferred embodiment of the invention, the *vlmD* gene is expressed under the control of a strong promoter chosen among Ptrc, lambda promoter, Plac.

In another embodiment of the invention the *vlmD* gene may be modified to improve its expression in the host microorganism or to improve the enzyme activity of the translated protein.

According to the invention the microorganism of the invention produces L-valine. This microorganism may produce L-valine naturally or not. If the microorganism does not produce naturally L-valine, the genes involved in L-valine biosynthesis coding for acetolactate synthase, keto-acid reductoisomerase, dihydroxy-acid dehydratase and branched chain amino acid transaminase are introduced in the microorganism.

In a preferred aspect of the invention, the microorganism used is a naturally producing L-valine microorganism. Preferentially, the microorganism belongs to a family selected from the group comprising *Enterobacteriaceae, Clostridiaceae, Bacillaceae, Streptomycetaceae, Corynebacteriaceae* and *Saccharomycetaceae.* More preferentially the microorganism is a species of *Escherichia, Clostridium, Bacillus, Klebsiella, Pantoea, Salmonella, Corynebacterium* or *Saccharomyces.* Even more preferentially the microorganism is either the species *Escherichia coli or Corynebacterium glutamicum or Clostridium acetobutylicum or Bacillus subtilis* or *Saccharomyces cerevisiae.*

According to a preferred aspect of the invention, the recombinant microorganism used in the process of production of isobutylamine is further modified to optimize its production of intermediates involved in isobutylamine biosynthesis such L-valine and pyruvate. For optimizing L-valine production, the microorganism is modified to overexpress at least one gene of the L-valine biosynthesis pathway, chosen among genes coding for: acetolactate synthase also named acetohydroxy acid synthase (*ilvl, ilvH, ilvN* and *ilvB* in *E. coli* or homologous genes), keto-acid reductoisomerase also named acid isomeroreductase (*ilvC* in *E. coli* or homologous gene), dihydroxy-acid dehydratase (*ilvD* in *E. coli* or homologous gene), branched chain amino acid transaminase (*ilvE* in *E. coli* or homologous gene) and leucine regulatory protein (*Irp* in *E. coli* of homologous gene).

Alternatively or in combination, the genes coding for the L-valine transporter *ygaZ and ygaH* or homologous are deleted in the microorganism of the invention. In another embodiment of the invention, *ilvN* gene is modified so as to produce an IIvN protein which is feedback deregulated. Such mutations of *ilvN* are disclosed in Park *et al.* (2011).

For optimizing pyruvate production, the microorganism is modified to overexpress at least one gene involved in pyruvate biosynthesis pathway, chosen among gene coding for phosphoglycerate mutase (*gpmA* and *pgml* in *E. coli* or homologous gene), enolase (*eno* in *E. coli* or homologous gene) or pyruvate kinase (*pykA* and *pykF* in *E. coli* or homologous gene). Alternatively or in combination, at least one gene involved in pyruvate degradation pathway is attenuated. This gene is chosen among pyruvate oxidase (*poxB* in *E. coli* or homologous gene), phosphate acetyltransferase (*pta* in *E. coli* or homologous gene), acetate kinase (*ackA* in *E. coli* or homologous gene), aldehyde/alcohol dehydrogenase (*adhE* in *E. coli* or homologous gene) or lactate dehydrogenase (*IldD, Idha* or *dld* in *E. coli* or homologous gene).

In a preferred embodiment of the invention the method for producing isobutylamine, and then isobutene, involves a recombinant microorganism containing the following genetic modifications:
- overexpression of gene coding for acetolactate synthase (*ilvN* and *ilvB*), keto-acid reductoisomerase (*ilvC*), dihydroxy-acid dehydratase (*ilvD*) and branched chain amino acid transaminase (*ilvE*) and for leucine regulatory protein (*Irp*) and valine decarboxylase (*vlmD*),
- inactivation of *ygaZ* and *ygaH* genes coding for valine transporters.

In the invention, an especially preferred simple carbon source is glucose. Another preferred simple carbon source is sucrose.

In a specific embodiment of the invention the produced isobutylamine may be isolated from the culture medium by evaporation or by other means known by the man skilled in the art.

### Isobutylamine conversion into isobutene

The present invention relates to a process for producing isobutene using isobutylamine as starting material, comprising carrying out a reaction of chemical conversion of an amine to an olefin.

The conversion of an amine to an olefin is characterized by the elimination of the nitrogen residue and the formation of a double bond carbon-to-carbon.

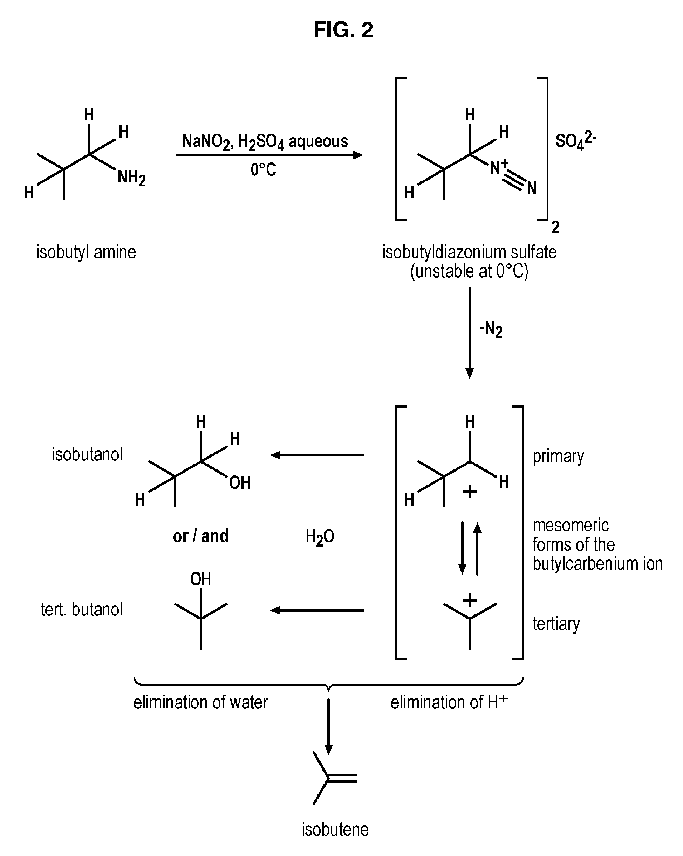

In a specific aspect of the invention, the chemical conversion of isobutylamine into isobutene is performed as a reaction of diazotation (figure 2), comprising:
a) a first step of treatment of isobutylamine with nitrous acid at 0°C,
b) a second step of loss of nitrogen resulting in the formation of mesomeric butyl-carbenium ion, and
c) a third step of elimination

Nitrous acid (HNO2 or HONO) reacts with primary aliphatic acid to give a clear solution and then nitrogen gas. Nitrous acid is a BrØnsted acid of moderate strength (pKa = 3.3). It is unstable, therefore it may be prepared immediately before use in the following manner:
NaNO2 + H2SO4 → H-O-N=O + NaHSO4

As shown in figure 2, the third step of elimination can be either:
- a step of proton elimination of the mesomeric butyl-carbenium ion, or
- a step of water elimination from the derived alcohols issued from the mesomeric butyl-carbenium ion.

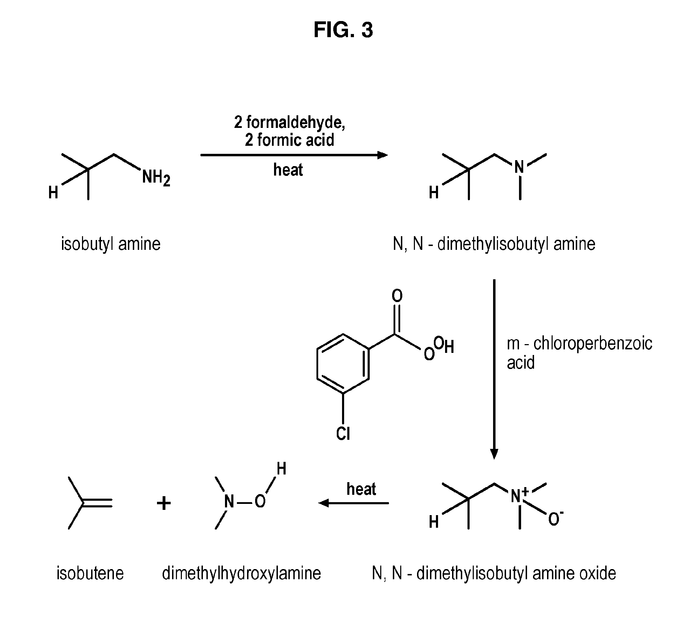

In another aspect of the invention, the chemical conversion of isobutylamine into isobutene is a 'Cope reaction' (figure 3), comprising:
a) a first step of preparation of a tertiary amine,
b) a second step of preparation of an amine oxide in presence of m-chloroperbenzoic acid, and
c) a third step of cleavage under heat into an olefin and a hydroxylamine derivative

The Cope reaction can be summarized as follow: from a tertiary amine, an amine oxide
is prepared by the action of m-chloroperbenzoic acid. In a next step the amine oxide is cleaved under the influence of heat into an olefin and a hydroxylamine derivative as a side product.

In the case of isobutyl amine the tertiary amine has to be prepared first. The method of the choice in such a situation is to use the Leuckart reaction. This is by far the most selective and technically feasible method for the production of amines. The Leuckart reaction can be easily and cleanly stopped at the stage of the desired tertiary amine.

In a specific aspect of this invention, the first step of preparation of a tertiary amine is the Leuckart reaction, performed in presence of formaldehyde and formic acid under heat.

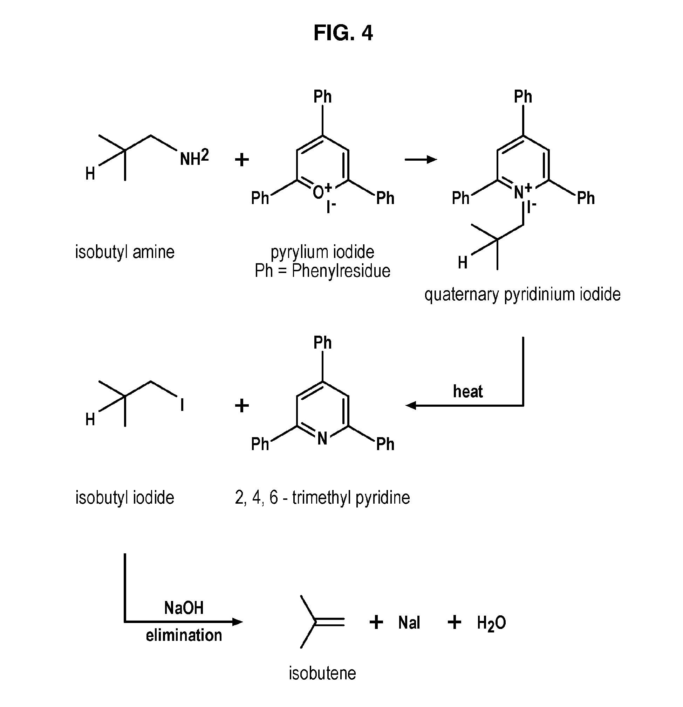

In a third aspect of the invention, the chemical conversion of isobutylamine into isobutene uses pyrylium salts, and comprises:
a) a first step of production of a quaternary pyridinium iodide, carried out in presence of 2,3,4-triphenyl-pyrylium iodide,
b) a second step of thermal elimination by heating to obtain isobutyliodide, and
c) a third step of elimination of hydrogen iodide with sodium hydroxide as a base (figure 4).

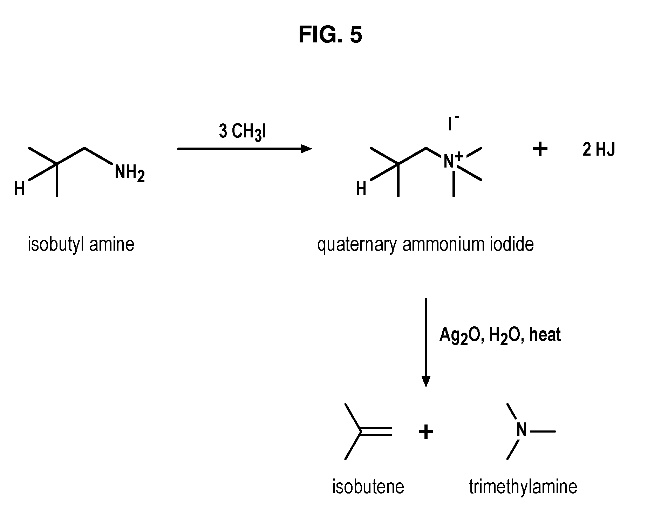

In a last aspect of the invention, the chemical conversion of isobutylamine into isobutene is a Hofmann elimination (figure 5), comprising:
a) a first reaction carried out in the presence of methyl iodide as catalyst,
b) a second reaction carried out in presence of silver oxide, water and heat, and
c) a third step of elimination.

In a preferred aspect of the invention, the gaseous isobutene is condensed in a cold trap after its synthesis in the reaction medium.

### DRAWINGS

Figure 1: Metabolic pathway for biosynthesis of isobutylamine.
Figure 2: Scheme of the diazotation of ethanolamine into isobutene
Figure 3: Scheme of the conversion of ethanolamine into isobutene by Cope reaction
Figure 4: Scheme of the conversion of ethanolamine into isobutene with pyrylium salts
Figure 5: Scheme of the conversion of ethanolamine into isobutene by Hofmann elimination

### EXAMPLES

The present invention is further defined in the following examples. It should be understood that these example, while indicating preferred embodiments of the invention, are given by way of illustration only. From above disclosure and these examples, the man skilled in the art can make various changes of the invention to adapt it to various uses and conditions without modify the essentials means of the invention.

In particular, examples show modified *Escherichia coli* (*E. coli*) strains, but these modifications can easily be performed on other microorganisms of the same family.

*E. coli* belongs to the *Enterobacteriaceae* family, which comprises members that are Gram-negative, rod-shaped, non-spore forming and are typically 1-5 µm in length. Most members have flagella used to move about, but a few genera are non-motile. Many members of this family are a normal part of the gut flora found in the intestines of humans and other animals, while others are found in water or soil, or are parasites on a variety of different animals and plants. *E. coli* is one of the most important model organisms, but other important members of the *Enterobacteriaceae* family include *Klebsiella,* in particular *Klebsiella terrigena, Klebsiella planticola* or *Klebsiella oxytoca,* and *Salmonella.*

### PROTOCOLES

Several protocols have been used to construct isobutylamine producing strain and are described in the following examples.

### Protocol 1: Chromosomal modification by homologous recombination and selection of recombinants (Datsenko & Wanner, 2000)

Allelic replacement or gene disruption in specified chromosomal locus is carried out by homologous recombination as described by Datsenko & Wanner (2000). The kanamycin (Km) resistance *kan* genes, flanked by Flp recognition sites, is amplified by PCR by using pKD4 plasmid as template. The resulting PCR products are used to transform the recipient *E. coli* strain harbouring plasmid pKD46 which expresses the system λ Red (y, β, exo) recombinase. The kanamycin-resistant transformants are then selected and the chromosomal structure of the mutated loci is verified by PCR analysis with the appropriate primers.

The *kan*-resistance gene is removed by using plasmid pCP20 as described by Datsenko & Wanner (2000). Antibiotic sensitive clones are then verified by PCR using specific primers.

### EXAMPLE 1

Calculation of maximum yields for isobutylamine production on glucose and sucrose

### 1.1 - Parameters used for simulations

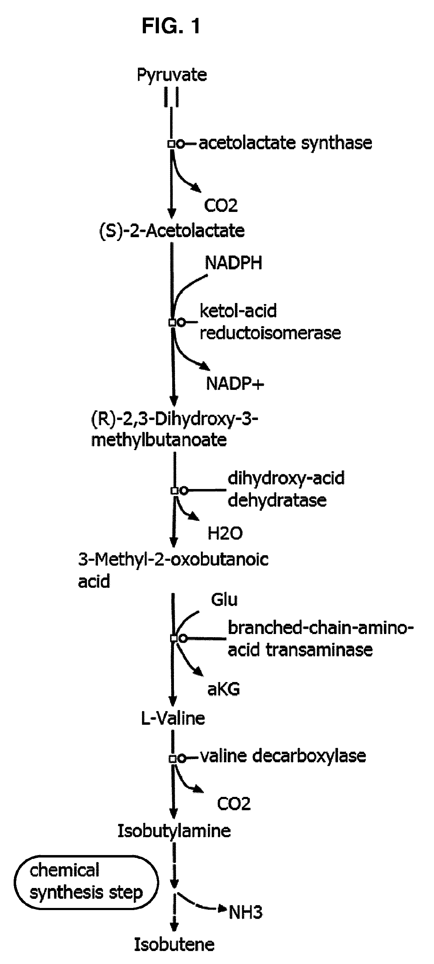

Simulations were performed with the METEX proprietary software METOPT™. A simplified metabolic network of *E. coli* was used including a central metabolic network, metabolic pathways for all biomass precursors and specific production pathway as described above and presented in Figure 1. A classical biomass composition for *E. coli* was used. Simulations were performed using either glucose or sucrose carbon source. For sucrose utilization, both the PTS system and the non-PTS system were modelled. As there were no differences on maximal yields calculated, only one yield on sucrose is reported. Calculation of a theoretical maximum yield was performed, taking into account no growth and no maintenance. Calculation of a practical maximum yield was performed, taking into account a growth rate of 0.1h⁻¹ and maintenance energy of 5 mmol_{ATP}.g_{DW}⁻¹.h⁻¹. All simulations were performed with a specific uptake rate of glucose of 3 mmol.g_{DW}⁻¹.h⁻¹, or a specific uptake rate of sucrose of 1.5 mmol.g_{DW}⁻¹.h⁻¹. Simulations were performed under aerobic conditions.

### 1.2 - Simulation results

| | isobutylamine on glucose | isobutylamine on sucrose |
|---|---|---|
| Maximum theoretical yield (g/g) | 0.40 | 0.43 |
| Maximum practical yield (g/g) | 0.27 | 0.29 |

The results show that integration of *vlmD* into *E. coli* allows the production of isobutylamine.

### EXAMPLE 2

Construction of strain *MG1655 ΔygaZH::Km (pCL1920-Ptrc01*/*RBS01*2-ilvBN*(GMV20-22DDF)C-P_{ilvE}-ilvED-TT07*) *(pUC18-Ptrc01*/*RBS01*2-vlmDsvO1ec-lrp-TT07)*

### 2.1 - Construction of strain MG1655 ΔygaZH::Km

To delete the *ygaZH* genes, which code for a L-valine exporter, in the strain MG1655 pKD46, Protocol 1 is used except that primers DygaZH-F (SEQ ID N°1) and DygaZH-R (SEQ ID N°2) are used to amplify the kanamycin resistance cassette from pKD4 plasmid. DygaZH-F (SEQ ID N°1) 
ATGGAAAGCCCTACTCCACAGCCTGCTCCTGGTTCGGCGACCTTCATGGAAGGATG
CAAAGACAGTTTACCGATTGTTATCATATGAATATCCTCCTTAG
 with
- sequence (upper case) homologous to sequence of *ygaZ* gene (2807639-2807718, reference sequence on the website http://ecogene.org/)
- sequence (bold upper case) corresponding to the primer site 2 of pKD4 plasmid (Datsenko & Wanner, 2000)

DygaZH-R (SEQ ID N°2) 
TTATATAATCGCCATCACTTTCCAGGCGAGCCCATAGGCCAGCGCACTAAGCAGTGT
TGGGATGATAATGCTGCGTGTTTTGTAGGCTGGAGCTGCTTCG
- sequence (upper case) homologous to sequence of *ygaH* gene (2808622-2808701, reference sequence on the website http://ecogene.org/)
- sequence (bold upper case) corresponding to the primer site 1 of pKD4 plasmid (Datsenko & Wanner, 2000)

Kanamycin resistant recombinants are selected. The insertion of the resistance cassette is then verified by PCR with primers ygaZF (SEQ ID N°3) and ygaHR (SEQ ID N°4) and by DNA sequencing. The verified and selected strain is called MG1655 *ΔygaZH::Km* pKD46.

ygaZF (SEQ ID N°3) GCCTGGCAACATGGCGGTTGGGC homologous to sequence upstream *ygaZ* gene (2807405-2807427, reference sequence on the website http://ecogene.org/)

ygaHR (SEQ ID N°4) CCTGAGACTCCAGCGTAATCAACGCC homologous to sequence downstream *ygaH* gene (2808968-2808993, reference sequence on the website http://ecogene.org/)

The thermo-sensitive pKD46 plasmid is removed by cultivating the strain MG1655 *ΔygaZH::Km* pKD46 at 42°C following by an isolation of the strain's culture on LB plates. Single clones are verified for the loss of ampicillin resistance and the verified and selected strain is called MG1655 *ΔygaZH::Km.*

Optionally the kanamycin resistance cassette could be removed according to Protocol 1.

### 2.2 - Construction of strain MG1655 ΔygaZH::Km (pCL1920-Ptrc01/RBS01*2-ilvBN*(GMV20-22DDF)C-P_{ilvE}-ilvED-TT07)

In order to overexpress the valine biosynthesis pathway, the pCL1920-P*trc01*/*RBS01*2-ilvBN*(GMV20-22DDF)*C-P*_{ilvE}ilvED-TT07* plasmid is constructed, which is derived from plasmid pCL1920 (Lerner & Inouye, 1990) and *ilvBN* operon coding for acetolactate synthase isoenzymes I, *ilvC* gene coding for ketoacid reductoisomerase and *ilvED* operon coding for branched-chain amino acid transaminase and dihydroxy acid dehydratase respectively.

In this plasmid, *ilvBN* and *ilvC* genes are expressed in an operon and their expression is driven by a constitutive *trc* promoter. The expression of the *ilvDE* operon is driven by its natural promoter corresponding to the -200 to -1 region upstream of the *ilvE* start codon.

### 2.2.1 - Construction of pCL1920-Ptrc01/RBS01*2-ilvBN*(GMV20-22DDF) plasmid

To construct the P*trc01*/*RBS01*2-ilvBN*(GMV20-22DDF)* insert, overlapping PCR between the *trc* promoter, the Ribosome Binding Site (RBS) sequence consensus and the *ilvBN* operon, in which six mutated bases (C59T, G60A, A62T, T63G, T64A, C66G) in *ilvN* gene are introduced into the oligonucleotides primers to generate the *ilvN*(GMV20-22DDF)* mutant, are used. The mutant *ilvN* described above confers L-valine feedback deregulation in *E. coli.*

First, the *trc* promoter and RBS consensus sequence, the *ilvB* gene and the beginning of *ilvN* gene are amplified from *E. coli* MG1655 genomic DNA by PCR using primers Ptrc01/RBS01*2-ilvB-KpnI-F (SEQ ID N°5) and ilvN*(GMV20-22DDF) R (SEQ ID N°6). Then, the end of *ilvN* gene is amplified from *E. coli* MG1655 genomic DNA by PCR using primers ilvN*(GMV20-22DDF) F (SEQ ID N°7) and ilvN BamHI R (SEQ ID N°8). Primers ilvN*(GMV20-22DDF) R (SEQ ID N°6) and ilvN*(GMV20-22DDF) F (SEQ ID N°7) are designed to overlap completely to each other. Finally, the Ptrc01/RBS01*2-*ilvBN*(GMV20-22DDF)* fragment is amplified by mixing the *trc* promoter-RBS-ilvBN' and the *ilvN'* amplicons using primers Ptrc01/RBS01*2-ilvB-KpnI-F (SEQ ID N°5) and ilvN BamHI R (SEQ ID N°8). The resulting fusion PCR product is cloned between the *KpnI* and *BamHI* sites of pCL1920 plasmid. The resulting plasmid is verified by DNA sequencing and called pCL1920-P*trc01*/*RBS01*2-ilvBN*(GMV20-22DDF).*

Ptrc01/RBS01*2-ilvB-KpnI-F (SEQ ID N°5) 
CGGGGTACCGAGCTGTTGACAATTAATCATCCGGCTCGTATAATGTGTGGAAGTCG
ACGTTAACCCTAGGTAAGGAGGTTATAAatggcaagttcgggcacaac
 with
- sequence (upper case) for *KpnI* restriction site and extrabases
- sequence (bold upper case) corresponding to *trc* promoter (Amann *et al.,* 1983 and Amann *et al.,* 1988)
- sequence (underlined upper case) for *Sal*I, *Hpa*I*, Avr*II restriction sites,
- sequence (italic upper case) corresponding to RBS consensus sequence with a *Psi*I restriction site
- sequence (lower case) homologous to the *ilvB* sequence (3850807-3850788, reference sequence on the website http://ecogene.org/)

ilvN*(GMV20-22DDF) R (SEQ ID N°6) ACAAACGTGGGT**g**A**agt**C**at**CCGGATGGTT homologous to the *ilvN* gene (3849038-3849067, reference sequence on the website tttp://ecogene.org/) containing six mutated bases (G59A, C60T, T62A, A63C, A64T, G66C) in order to generate 3 amino-acid substitution G20D, M21 D and V22F.

ilvN*(GMV20-22DDF) F (SEQ ID N°7) AACCATCCGG**at**G**act**T**c**ACCCACGTTTGT homologous to the *ilvN* gene (3849038-3849067, reference sequence on the website http://ecogene.org/) containing six mutated bases (C59T, G60A, A62T, T63G, T64A, C66G, bold lower case) in order to generate 3 amino-acid substitution G20D, M21 D and V22F.

ilvN BamHI R (SEQ ID N°8) GCGGGATCCTACGTATTAATTAA**TTACTGAAAAAACACCGCGATC**
with
- sequence for *Bam*HI, SnaBI and *Pac*I restriction sites and extrabases (upper case)
- sequence (bold upper case) homologous to the *ilvN* sequence (3848825-3848846, reference sequence on the website http://ecogene.org/)

### 2.2.2 - Construction of pCL1920-Ptrc01/RBS01*2-ilvBN*(GMV20-22DDF)C plasmid

To construct the *pCL1920-Ptrc01*/*RBS01*2-ilvBN*(GMV20-22DDF)C,* the *ilvC* gene is amplified from *E. coli* MG1655 genomic DNA by PCR using primers ilvC Pacl F (SEQ ID N°9) and ilvC SnaBI R (SEQ ID N°10). The obtained fragment is digested by *Pac*I and *Sna*BI and ligated into the *Pac*I/*SnaB*I digested pCL1920-P*trc*01/*RBS01***2-ilvBN*(GMV20-22DDF)* plasmid. The resulting plasmid is verified by DNA sequencing and called pCL1920-P*trc01*/*RBS01*2-ilvBN*(GMV20-22DDF)C.*

ilvC PacI F (SEQ ID N°9) ccattaattaa**ACGAGGAATCACC**ATGGCTAAC
- sequence (lower case) for *Pac*I restriction site and extrabases
- sequence (bold upper case) homologous to the putative RBS sequence of *ilvC* gene (3955980-3955992, reference sequence on the website http://ecogene.org/)
- sequence (upper case) homologous to the beginning of *ilvC* gene (3955993-3956001, reference sequence on the website http://ecogene.org/)

ilvC SnaBI R (SEQ ID N°10) GCGTACGTAGCTAGC**TTAACCCGCAACAGCAATAC**
- sequence (upper case) for *SnaB*I and *Nhe*l restriction sites and extrabases
- sequence (bold upper case) homologous to the end of *ilvC* gene (3957449-3957468, reference sequence on the website http://ecogene.org/)

### 2.2.3 - Construction of pCL1920-Ptrc01/RBS01*2-ilvBN*(GMV20-22DDF)C-PilvE-ilvED-TT07 plasmid

To construct the plasmid pCL1920-P*trc01*/*RBS01*2-ilvBN*(GMV20-22DDF*)C-P*_{ilvE}*-*ilvE*D-TT07, the *ilvED* operon and its natural promoter are amplified from *E. coli* MG1655 genomic DNA by PCR using primers PilvE NheI F (SEQ ID N°11) and ilvD-TT07-Xbal R (SEQ ID N°12). The obtained fragment is digested by *Nhe*I and *Xbal*I and ligated into the *Nhe*I/*Xba*I digested pCL1920-Ptrc01/RBS01*2-*ilvBN***(GMV20-22DDF*)C plasmid. The resulting plasmid is verified by DNA sequencing and called pCL1920-P*trc01*/*RBS01*2-ilvBN*(GMV20-22DDF*)*C-*P*_{ilvE}-ilvE*D-*TT07.*

PilvE NheI F (SEQ ID N°11) ctgagctagcTTTCCACGTCTGCTCAATGA
- sequence (lower case) for *Nhe*I restriction site and extrabases
- sequence (upper case) homologous to the *ilvE* promoter region (3950307-3950326, reference sequence on the website http://ecogene.org/)

ilvD-TT07-Xbal R (SEQ ID N°12) 
TAGTCTAGAGCAGAAAGGCCCACCCGAAGGTGAGCCAGtacgtaTTAACCCCCAGTT
TCGATT
- sequence (upper case) for *Xba*I restriction site and extrabases
- sequence (bold upper case) corresponding to T7Te transcriptional terminator sequence from T7 phage (Harrington *et al.,* 2001).
- sequence (lower case) corresponding to *SnaB*I restriction site
- sequence (underlined upper case) homologous to the end of *ilvD* gene (3953332-3953351, reference sequence on the website http://ecogene.org/)

### 2.2.4 - Construction of the strain MG1655 ΔygaZH::Km (pCL1920-Ptrc01/RBS01*2-ilvBN*(GMV20-22DDF)C-PilvE-ilvED-TT07)

The plasmid pCL1920-P*trc01*/*RBS01*2-ilvBN*(GMV20-22DDF*)C-P*_{ilvE}-ilvE*D-*TT07,* constructed above, is introduced by electroporation into strain MG1655 *ΔygaZH::Km.* The presence of plasmid pCL1920-P*trc01*/*RBS01*2-ilvBN*(GMV20-22DDF)C*-P*_{ilvE}-ilvE*D-TT07 is verified and the resulting strain is called MG1655 *ΔygaZH::Km* (pCL1920-*Ptrc01*/*RBS01*2-ilvBN*(GMV20-22DDF)C*-P*_{ilvE}-ilvE*D-*TT07*)*.*

### 2.3 - Construction of strain MG1655 ΔygaZH::Km (pCL1920-Ptrc01/RBS01*2-ilvBN*1C-PilvE-ilvED-TT07) (pUC18-Ptrc01/RBS01*2-vlmDsvI1ec-Irp-TT07)

### 2.3.1 - Synthetic gene vlmDsvO1ec

A *vlmD* synthetic gene of *Streptomyces viridifaciens* coding for a valine decarboxylase is synthesized by Invitrogen. The codon usage and GC content of the gene is adapted to *E. coli* according to the supplier's matrix. The synthesized fragment is cloned into supplier's pM vectors and verified by sequencing.

The *vlmD* gene sequence from *S. viridifaciens* (AY116644) optimized for *Escherichia coli : vlmDsvO1ec* contains the following sequences: 
ATGAGCACCAGCAGCGCAAGCAGCGGTCCGGATCTGCCGTTTGGTCCGGAAGATAC
CCCGTGGCAGAAAGCATTTAGCCGTCTGCGTGCAGTTGATGGTGTTCCGCGTGTTAC
CGCACCGAGCAGCGATCCGCGTGAAGTTTATATGGATATTCCGGAAATTCCGTTCAG
CAAAGTTCAGATTCCGCCTGATGGTATGGATGAACAGCAGTATGCAGAAGCAGAAAG
CCTGTTTCGTCGTTATGTTGATGCACAGACCCGTAATTTTGCAGGTTATCAGGTTACC
AGCGATCTGGATTATCAGCATCTGAGCCATTATCTGAATCGCCATCTGAATAATGTTG
GCGATCCGTATGAAAGCAGCAGCTATACCCTGAATAGCAAAGTGCTGGAACGTGCA
GTGCTGGATTATTTTGCAAGCCTGTGGAATGCAAAATGGCCTCATGATGCAAGCGAT
CCGGAAACCTATTGGGGTTATGTTCTGACAATGGGTAGCAGCGAAGGTAATCTGTAT
GGTCTGTGGAACGCACGTGATTATCTGAGCGGTAAACTGCTGCGTCGTCAGCATCGT
GAAGCCGGTGGTGATAAAGCAAGCGTTGTTTATACCCAGGCACTGCGTCATGAAGGT
CAGAGTCCGCATGCCTATGAACCGGTTGCATTTTTTAGCCAGGATACCCATTATAGC
CTGACCAAAGCAGTTCGTGTTCTGGGTATTGATACCTTTCATAGCATTGGTAGCAGCC
GTTATCCGGATGAAAATCCGCTGGGTCCGGGTACACCGTGGCCGACCGAAGTTCCG
AGCGTGGATGGTGCAATTGATGTTGATAAACTGGCAAGCCTGGTTCGTTTTTTTGCCA
GCAAAGGTTATCCGATTCTGGTGAGCCTGAATTATGGTAGCACATTTAAAGGTGCCTA
TGATGATGTTCCGGCAGTTGCACAGGCCGTTCGTGATATTTGTACCGAATATGGTCT
GGATCGTCGTCGTGTTTATCATGATCGTAGCAAAGATAGCGATTTTGATGAACGTAGC
GGCTTTTGGATTCATATTGATGCAGCACTGGGTGCCGGTTATGCACCGTATCTGCAA
ATGGCACGTGATGCAGGTATGGTTGAAGAAGCACCGCCTGTTTTTGATTTTCGTCTG
CCGGAAGTTCATAGTCTGACCATGAGCGGTCATAAATGGATGGGCACCCCGTGGGC
ATGTGGTGTTTATATGACCCGTACTGGCCTGCAAATGACCCCTCCGAAAAGCAGCGA
ATATATCGGTGCAGCAGATACCACCTTTGCAGGTAGCCGTAATGGTTTTAGCAGCCT
GCTGCTGTGGGATTATCTGTCACGTCATAGTTATGATGATCTGGTTCGTCTGGCAGC
AGATTGTGATCGCCTGGCAGGTTATGCCCATGATCGTCTGCTGACCCTGCAAGACAA
 
ACTGGGCATGGATCTGTGGGTTGCACGTAGTCCGCAGAGCCTGACCGTTCGTTTTC
GTCAGCCGTGTGCAGATATTGTTCGTAAATATAGCCTGTCATGCGAAACCGTGTATG
AAGATAATGAACAGCGTACCTATGTTCATCTGTATGCAGTTCCGCATCTGACCCGTGA
ACTGGTTGATGAACTGGTGCGTGATCTGCGTCAGCCTGGTGCATTTACCAATGCCGG
TGCACTGGAAGGTGAAGCATGGGCAGGCGTTATTGATGCCCTGGGTCGTCCTGATC
CGGATGGCACCTATGCGGGTGCCCTGAGCGCACCGGCAAGTGGTCCGCGTAGCGA
AGATGGTGGTGGTAGCTAAGGATCCGAGCTCGAATTC
- sequence (upper case) corresponding to the *vlmD* optimised gene
- sequence (underlined upper case) corresponding to *BamH*I, *Sac*I and *EcoR*I restriction sites.

### 2.3.2 - Construction of pUC18-Ptrc01/RBS01*2-vlmDsvO1ec plasmid

In order to overexpress the valine decarboxylase to convert valine to isobutylamine, the plasmid pUC18-P*trc01*/*RBS01*2-vlmDsvO1ec* is constructed, which is derived from plasmid pUC18 (Norrander *et al.,* 1983) and the *vlmD* synthetic gene, described above, from *Streptomyces viridifaciens,* whose expression is driven by a constitutive *trc* promoter.

To construct the pUC18-Ptrc01/RBS01*2-*vlmDsvO1ec*, the P*trc01*/*RBS01*2-vlmDsvO1ec* fragment is amplified from the pM vector harbouring the *vlmDsvO1ec* synthetic gene providing by the supplier by PCR using primers Ptrc01/RBS01*2-vlmDsvO1ec Hindlll F (SEQ ID N°13) and vlmDsvO1ec BamHI R (SEQ ID N°14) and the obtained fragment is digested by *Hind*III and *BamH*I and ligated into the *Hind*III/*BamH*I digested pUC18 plasmid. The resulting plasmid is verified by DNA sequencing and called pUC18-P*trc01*/*RBS01*2-vlmDsvO1ec.*

Ptrc01/RBS01*2-vlmDsvO1ec Hindlll F (SEQ ID N°13) 
cccaagcttGAGCTGTTGACAATTAATCATCCGGCTCGTATAATGTGTGGAAgtcgacgttaa
cacgcgtTTTAAATAAGGAGGTTATAAATGAGCACCAGCAGCGCAAG
- sequence (lower case) for *Hind*III restriction site and extrabases
- sequence (bold upper case) corresponding to *trc* promoter (Amann *et al.,* 1983 and Amann *et al.,* 1988)
- sequence (italic lower case) for *Sal*I, *Hpa*I and *Mlu*I restriction sites,
- sequence (italic upper case) corresponding to RBS consensus sequence with a *Psi*I restriction site
- sequence (underlined upper case) corresponding to the beginning of the synthetic *vlmDsvO1ec* sequence gene.

vlmDsvO1ec BamHI R (SEQ ID N°14) GAATTCGAGCTCGGATCC**TTAGCTACCACCACCATCTTC**
- sequence (upper case) for *EcoR*I, *SacI*I, *BamH*I restriction sites,
- sequence (bold upper case) homologous to the end of the synthetic *vlmDsvO1ec* sequence gene.

### 2.3.3 - Construction of pUC18-Ptrc01/RBS01*2-vlmDsvO1ec-Irp-TT07 plasmid

In order to overexpress the leucine regulatory protein encoded by *Irp* gene, the pUC18-P*trc01*/*RBS01*2-vlmDsvO1ec-Irp-TT07* is constructed, which is derived from pUC18-P*trc01*/*RBS01*2-vlmDsvO1ec* plasmid described above and *Irp* gene from *E. coli* which is expressed in operon with *vlmDsvO1ec.*

To construct the pUC18-P*trc01*/*RBS01*2-vlmDsvO1ec-Irp-TT07,* the *Irp* and a part of its 5' untranslated region containing the RBS sequence *(*corresponding to the -59 to -1 region upstream of the *Irp* start codon) fragment are amplified from *E. coli* MG1655 genomic DNA by PCR using primers Irp BamHI F (SEQ ID N°15) and Irp-TT07 KpnI R (SEQ ID N°16) and the obtained fragment is digested by *BamHI* and *Kpn*I and ligated into the *BamH*I/*Kpn*I digested pUC18-Ptrc01/RBS01*2-*vlmDsvO1ec* plasmid. The resulting plasmid is verified by DNA sequencing and called pUC18-P*trc01*/*RBS01*2-vlmDsvO1ec-Irp-TT07.*

Irp BamHI F (SEQ ID N°15) cgcggatccGAACAGTGATGTTTCAGGGTC
- sequence (lower case) for *BamH*I restriction site and extrabases
- sequence (upper case) corresponding to the 5' untranslated region of *Irp* gene (931759-931779, reference sequence on the website http://ecogene.org/).

Irp-TT07 Kpnl R (SEQ ID N°16) 
cgggtaccGCAGAAAGGCCCACCCGAAGGTGAGCCAGTTAGCGCGTCTTAATAACCAG
AC
- sequence (lower case) for *Kpn*I restriction site and extrabases
- sequence (upper case) corresponding to T7Te transcriptional terminator sequence from T7 phage (Harrington *et al.,* 2001),
- sequence (bold upper case) homologous to the end of *Irp* gene (932290-932313, reference sequence on the website http://ecogene.org/)

### 2.3.4 - Construction of the strain MG1655 ΔygaZH::Km (pCL1920-Ptrc01/RBS01*2-ilvBN*(GMV20-22DDF)C-P_{ilvE}-ilvED-TT07) (pUC18-Ptrc01/RBS01*2-vlmDsvO1ec-Irp-TT07)

The plasmid pUC18-P*trc01*/*RBS01*2-vlmDsvO1ec-Irp-TT07,* constructed above, is introduced by electroporation into strain MG1655 Δ*ygaZH::Km* (pCL1920-P*trc01*/*RBS01*2-ilvBN*(GMV20-22DDF)C*-P*_{ilvE}-ilvED*-TT07). The presence of plasmid pUC18-P*trc01*/*RBS01*2-vlmDsvO1ec-Irp-TT07* is verified and the resulting strain is called MG1655 *ΔygaZH::Km* (pCL1920-P*trc01*/*RBS01*2-ilvBN*(GMV20-22DDF)*C-P*_{ilvE}-ilvE*D-TT07) (pUC18-P*trc01*/*RBS01*2-vlmDsvO1ec-Irp-TT07*).

### EXAMPLE 3

Culture of above described isobutylamine production strain on glucose

The production strain is evaluated in small Erlenmeyer flasks using modified M9 medium (Anderson, 1946) that is supplemented with 0.03 g.L⁻¹ of pyridoxine, 10 g.L⁻¹ MOPS and 10 g.L⁻¹ glucose and adjusted to pH 6.8.

A 5 mL preculture is grown at 37°C for 6.5 hours in a mixed medium (10 % LB medium (Sigma 25 %) with 2.5 g.L⁻¹ glucose and 90 % minimal medium described above). It is used to inoculate a 50 mL culture to an OD₆₀₀ of 0.1 in minimal medium. When necessary, antibiotics are added at concentrations of 50 mg.L⁻¹ for kanamycin, spectinomycin and ampicillin. The temperature of the cultures is 37°C. When the culture reaches an OD₆₀₀ of 7 to 9, extracellular metabolites are analyzed using HPLC with refractometric detection (organic acids and glucose). Production of isobutylamine is determined by GC/MS.

This strain produces isobutylamine with a concentration ranging between 0,001 and 100 mM.

### REFERENCES

- Amann E, Brosius J, Ptashne M (1983), Gene. 25:167-178
- Amann E, Ochs B, Abel KJ (1988), Gene. 69:301-315
- Anderson EH (1946), Proc Natl Acad Sci USA. 32:120-128
- Bast E, Hartmann T, Steiner M (2000), Arch Mikrobiol. 79:12-24
- Datsenko KA, Wanner BL (2000), Proc Natl Acad Sci USA. 97:6640-6645
- Garg RP, Ma Y, Hoyt JC, Parry RJ (2002), Mol Microbiol. 46:505-517
- Harrington KJ, Laughlin RB, Liang S (2001), Proc Natl Acad Sci USA. 98:5019-5024
- Haughton BG, King HK (1961), Biochem J. 80:268-277
- Lerner CG, Inouye M (1990), Nucleic Acids Res. 18:4631
- Liebl W, Klamer R, Schleifer KH (1989), ApplMicrobiol Biotechnol. 32:205-210
- Miller JH (1992), A Short Course in Bacterial Genetics: A Laboratory Manual and Handbook for Escherichia coli and Related Bacteria, Cold Spring Harbor Laboratory Press, Cold Spring Harbor, New York
- Norrander J, Kempe T, Messing J (1983), Gene. 26:101-106
- Park JH, Kim TY, Lee KH, Lee SY (2011), Biotechnol Bioeng. 108:934-946
- Prescott L et al. (1999), "Microbiology" 4th Edition, WCB McGraw-Hill;
- Riedel C, Rittmann D, Dangel P, Möckel B, Petersen S, Sahm H, Eikmanns BJ (2001), J Mol Microbiol Biotechnol. 3:573-583
- Sambrook J et al. (1989) (2001), "Molecular Cloning: A Laboratory Manual" 2nd & 3rd Editions, Cold Spring Harbor Laboratory Press
- Schaefer U, Boos W, Takors R, Weuster-Botz D (1999), Anal Biochem. 270:88-96

## Claims

1. A method for producing isobutene comprising:
- production of isobutylamine by fermentation in a culture medium comprising a simple source of carbon of a recombinant microorganism producing L-valine, and
- conversion of isobutylamine into isobutene by chemical conversion
wherein the recombinant microorganism expresses an exogenous gene coding for an enzyme having L-valine decarboxylase activity.

2. A method according to claim 1, wherein the exogenous gene coding for the enzyme having L-valine decarboxylase activity is the gene *vlmD* from *Streptomyces viridifaciens.*

3. A method according to anyone of claims 1 to 2, wherein the recombinant microorganism over-expresses at least one endogenous gene of the L-valine biosynthesis pathway.

4. A method according to claim 3, wherein the endogenous gene of the L-valine biosynthesis pathway is chosen among a gene coding for acetolactate synthase, keto-acid reductoisomerase, dihydroxy-acid dehydratase, branched chain amino acid transaminase and leucine regulatory protein.

5. A method according to anyone of claims 1 to 4, wherein the microorganism is from the family *Enterobacteriaceae* or *Saccharomycetaceae.*

6. A method according to claim 5, wherein the microorganism is from the species *Escherichia coli.*

7. A method according to anyone of claims 1 to 6, wherein the simple carbon source is chosen from the group consisting of: glucose or sucrose.

8. A method according to anyone of claims 1 to 7, wherein the produced isobutylamine is isolated from the culture medium.

9. The process according to claim 1, wherein the isobutylamine is chemically converted into isobutene by diazotation.

10. The process according to claim 1, wherein the isobutylamine is chemically converted into isobutene by a Cope reaction, comprising a first step of preparation of a tertiary amine.

11. The process according to claim 1, wherein the isobutylamine is chemically converted into isobutene by action of pyrylium salts.

12. The process according to claim 1, wherein the isobutylamine is chemically converted into isobutene by a Hofmann elimination.

13. A genetically modified microorganism overexpressing at least one endogenous gene involved in the L-valine biosynthesis pathway and expressing a gene coding for a L-valine decarboxylase.


SEQUENCE LISTING

<110>  METABOLIC EXPLORER
       Boisart, Cedric

<120>  PROCESS FOR PRODUCING ISOBUTENE FROM ISOBUTYLAMINE

<130>  D29552

<160>  17

<170>  PatentIn version 3.5

<210>  1
<211>  100
<212>  DNA
<213>  Artificial Sequence

<220>
<223>  Oligonucleotide

<400>  1
atggaaagcc ctactccaca gcctgctcct ggttcggcga ccttcatgga aggatgcaaa      60

gacagtttac cgattgttat catatgaata tcctccttag                            100


<210>  2
<211>  100
<212>  DNA
<213>  Artificial Sequence

<220>
<223>  Oligonucleotide

<400>  2
ttatataatc gccatcactt tccaggcgag cccataggcc agcgcactaa gcagtgttgg      60

gatgataatg ctgcgtgttt tgtaggctgg agctgcttcg                            100


<210>  3
<211>  23
<212>  DNA
<213>  Artificial Sequence

<220>
<223>  Oligonucleotide

<400>  3
gcctggcaac atggcggttg ggc                                               23


<210>  4
<211>  26
<212>  DNA
<213>  Artificial Sequence

<220>
<223>  Oligonucleotide

<400>  4
cctgagactc cagcgtaatc aacgcc                                            26

<210>  5
<211>  104
<212>  DNA
<213>  Artificial Sequence

<220>
<223>  Oligonucleotide

<400>  5
cggggtaccg agctgttgac aattaatcat ccggctcgta taatgtgtgg aagtcgacgt      60

taaccctagg taaggaggtt ataaatggca agttcgggca caac                      104


<210>  6
<211>  30
<212>  DNA
<213>  Artificial Sequence

<220>
<223>  Oligonucleotide

<400>  6
acaaacgtgg gtgaagtcat ccggatggtt                                      30


<210>  7
<211>  30
<212>  DNA
<213>  Artificial Sequence

<220>
<223>  Oligonucleotide

<400>  7
aaccatccgg atgacttcac ccacgtttgt                                      30


<210>  8
<211>  45
<212>  DNA
<213>  Artificial Sequence

<220>
<223>  Oligonucleotide

<400>  8
gcgggatcct acgtattaat taattactga aaaaacaccg cgatc                     45


<210>  9
<211>  33
<212>  DNA
<213>  Artificial Sequence

<220>
<223>  Oligonucleotide

<400>  9
ccattaatta aacgaggaat caccatggct aac                                  33


<210>  10
<211>  35
<212>  DNA

<213>  Artificial Sequence

<220>
<223>  Oligonucleotide

<400>  10
gcgtacgtag ctagcttaac ccgcaacagc aatac                                35


<210>  11
<211>  30
<212>  DNA
<213>  Artificial Sequence

<220>
<223>  Oligonucleotide

<400>  11
ctgagctagc tttccacgtc tgctcaatga                                      30


<210>  12
<211>  64
<212>  DNA
<213>  Artificial Sequence

<220>
<223>  Oligonucleotide

<400>  12
tagtctagag cagaaaggcc cacccgaagg tgagccagta cgtattaacc ccccagtttc      60

gatt                                                                  64


<210>  13
<211>  110
<212>  DNA
<213>  Artificial Sequence

<220>
<223>  Oligonucleotide

<400>  13
cccaagcttg agctgttgac aattaatcat ccggctcgta taatgtgtgg aagtcgacgt      60

taacacgcgt tttaaataag gaggttataa atgagcacca gcagcgcaag                110


<210>  14
<211>  39
<212>  DNA
<213>  Artificial Sequence

<220>
<223>  Oligonucleotide

<400>  14
gaattcgagc tcggatcctt agctaccacc accatcttc                            39


<210>  15
<211>  30
<212>  DNA
<213>  Artificial Sequence

<220>
<223>  Oligonucleotide

<400>  15
cgcggatccg aacagtgatg tttcagggtc                                        30


<210>  16
<211>  60
<212>  DNA
<213>  Artificial Sequence

<220>
<223>  Oligonucleotide

<400>  16
cgggtaccgc agaaaggccc acccgaaggt gagccagtta gcgcgtctta ataaccagac      60


<210>  17
<211>  1803
<212>  DNA
<213>  Artificial Sequence

<220>
<223>  Synthetic gene from Streptomyces viridifaciens, adapted to E.
       coli

<400>  17
atgagcacca gcagcgcaag cagcggtccg gatctgccgt ttggtccgga agataccccg      60

tggcagaaag catttagccg tctgcgtgca gttgatggtg ttccgcgtgt taccgcaccg     120

agcagcgatc cgcgtgaagt ttatatggat attccggaaa ttccgttcag caaagttcag     180

attccgcctg atggtatgga tgaacagcag tatgcagaag cagaaagcct gtttcgtcgt     240

tatgttgatg cacagacccg taattttgca ggttatcagg ttaccagcga tctggattat     300

cagcatctga gccattatct gaatcgccat ctgaataatg ttggcgatcc gtatgaaagc     360

agcagctata ccctgaatag caaagtgctg gaacgtgcag tgctggatta ttttgcaagc     420

ctgtggaatg caaaatggcc tcatgatgca agcgatccgg aaacctattg gggttatgtt     480

ctgacaatgg gtagcagcga aggtaatctg tatggtctgt ggaacgcacg tgattatctg     540

agcggtaaac tgctgcgtcg tcagcatcgt gaagccggtg gtgataaagc aagcgttgtt     600

tatacccagg cactgcgtca tgaaggtcag agtccgcatg cctatgaacc ggttgcattt     660

tttagccagg atacccatta tagcctgacc aaagcagttc gtgttctggg tattgatacc     720

tttcatagca ttggtagcag ccgttatccg gatgaaaatc cgctgggtcc gggtacaccg     780

tggccgaccg aagttccgag cgtggatggt gcaattgatg ttgataaact ggcaagcctg     840

gttcgttttt ttgccagcaa aggttatccg attctggtga gcctgaatta tggtagcaca     900

tttaaaggtg cctatgatga tgttccggca gttgcacagg ccgttcgtga tatttgtacc     960

gaatatggtc tggatcgtcg tcgtgtttat catgatcgta gcaaagatag cgattttgat    1020

gaacgtagcg gcttttggat tcatattgat gcagcactgg gtgccggtta tgcaccgtat    1080

ctgcaaatgg cacgtgatgc aggtatggtt gaagaagcac cgcctgtttt tgattttcgt 1140

ctgccggaag ttcatagtct gaccatgagc ggtcataaat ggatgggcac cccgtgggca 1200

tgtggtgttt atatgacccg tactggcctg caaatgaccc ctccgaaaag cagcgaatat 1260

atcggtgcag cagataccac ctttgcaggt agccgtaatg gttttagcag cctgctgctg 1320

tgggattatc tgtcacgtca tagttatgat gatctggttc gtctggcagc agattgtgat 1380

cgcctggcag gttatgccca tgatcgtctg ctgaccctgc aagacaaact gggcatggat 1440

ctgtgggttg cacgtagtcc gcagagcctg accgttcgtt ttcgtcagcc gtgtgcagat 1500

attgttcgta aatatagcct gtcatgcgaa accgtgtatg aagataatga acagcgtacc 1560

tatgttcatc tgtatgcagt tccgcatctg acccgtgaac tggttgatga actggtgcgt 1620

gatctgcgtc agcctggtgc atttaccaat gccggtgcac tggaaggtga agcatgggca 1680

ggcgttattg atgccctggg tcgtcctgat ccggatggca cctatgcggg tgccctgagc 1740

gcaccggcaa gtggtccgcg tagcgaagat ggtggtggta gctaaggatc cgagctcgaa 1800

ttc 1803